# EUROPÄISCHE PATENTANMELDUNG

(11) **EP 2 338 540 A1**
(43) Veröffentlichungstag der Anmeldung: **29.06.2011**
(21) Anmeldenummer: 09075571.1
(22) Anmeldetag: 23.12.2009
(51) Int. Cl.: A61M 1/10, F04D 29/18

(54) **Förderschaufel für einen komprimierbaren Rotor**

(71) Anmelder: ECP Entwicklungsgesellschaft mbH, 12247 Berlin (DE)
(72) Erfinder: Er, Sami, 10715 Berlin (DE)
(74) Vertreter: Pfenning, Meinig & Partner GbR

(57) **Zusammenfassung**

Um eine einfache, jedoch sehr flexibel handhabbare und komprimierbare Ausführung eines Rotors (2) für eine Fluidpumpe zu schaffen, wird gemäß der Erfindung eine Förderschaufel mit wenigstens zwei Streben (12, 13, 14) und einer im expandierten Zustand von diesen gehaltenen Membran geschaffen, bei der die Streben jeweils wenigstens ein, insbesondere mehr als ein Gelenk aufweisen, das in einer ersten Bewegungsebene in einer ersten Richtung ein Abwinkeln ermöglicht und in der entgegengesetzten zweiten Richtung ein Überstrecken über einen Streckwinkel von insbesondere 180° begrenzt. Insbesondere wenn mehrere Gelenke (15, 16, 17) an den Streben vorgesehen sind, werden diese und mit ihnen die Förderschaufel für eine einfache Komprimierbarkeit besonders flexibel.

## Beschreibung

Die Erfindung liegt auf dem Gebiet des Maschinenbaus, insbesondere der Mikromechanik, und bezieht sich auf Förderschaufeln für Rotoren von Fluidpumpen, die besonders geeignet zum Einsatz in schwierig zugänglichen Bereichen sind.

Solche Pumpen können beispielsweise in der Medizintechnik als Mikropumpen derart klein gestaltet sein, dass sie mikroinvasiv in Blutgefäße eingeführt werden können. Sie können dann zur Unterstützung der Pumpkraft des menschlichen Herzens eingesetzt werden.

Um derartige Pumpen vorteilhaft einsetzbar zu machen, sind sie oft komprimierbar und expandierbar gestaltet, so dass sie in einem komprimierten Zustand durch ein Blutgefäß beispielsweise bis in eine Herzkammer eingeführt und dort expandiert werden können.

Zu diesem Zweck ist es oft vorgesehen, dass ein entsprechender Rotor der Pumpe, der im Betrieb sehr schnell antreibbar ist, zwischen dem Transportzustand und dem Betriebszustand in der Größe radial veränderbar, d. h. komprimierbar und expandierbar ist.

Ähnliche Anwendungen sind jedoch auch im nichtmedizinischen Bereich bei größeren Pumpen denkbar, die in schwierig zugänglichen Bereichen eingesetzt werden sollen.

Eine Vielzahl solcher Pumpen ist bereits bekannt, wobei verschiedene Prinzipien verwendet werden, um eine Komprimierbarkeit zu erreichen. Es ist aus der WO 98/53864 bekannt, eine Axialpumpe mit einem Rotor einzusetzen, wobei der Rotor die Form eines starren Rohres aufweist, das außen in einem Stator gelagert ist. Der Antrieb kann dabei direkt in den Stator und den Rotor als elektromagnetischer Antrieb integriert sein.

Eine Pumpe mit komprimierbarem Rotor ist im Gegensatz dazu aus der WO 03/103745 A2 bekannt, wobei bei dieser Pumpe der Rotor ein entfaltbares Rotorblatt aufweist, das sich im Betrieb durch den Fluidgegendruck des Blutes entfaltet.

Der vorliegenden Erfindung liegt im Hinblick auf den Stand der Technik die Aufgabe zugrunde, einen Rotor bzw. eine Förderschaufel für einen Rotor zu schaffen, die komprimierbar und expandierbar sind und dabei konstruktiv möglichst einfach gestaltet, zuverlässig und haltbar sowie kostengünstig herstellbar sind.

Die Aufgabe wird mit den Merkmalen der Erfindung gemäß Patentanspruch 1 gelöst.

Dabei weist eine entsprechende Förderschaufel wenigstens zwei Streben sowie eine im expandierten Zustand des Rotors bzw. im gestreckten Zustand der Streben zwischen diesen gehaltene Membran auf, und die Streben weisen ihrerseits jeweils wenigstens ein, insbesondere mehr als ein Gelenk auf, das in einer ersten Bewegungsebene in einer Richtung ein Abwinkeln ermöglicht und in der entgegengesetzten zweiten Richtung innerhalb der ersten Bewegungsebene ein Überstrecken über einen Streckwinkel, insbesondere 180° begrenzt. Der Streckwinkel kann auch bei weniger als 180°, beispielsweise bei 170°, 160° oder 150° oder über 180°, beispielsweise bei 190°, 200° oder 210° liegen, wenn eine im Betriebszustand gekrümmte Strebe realisiert werden soll. Der Streckwinkel einander benachbarter Gelenke kann gleich sein, jedoch auch variieren. Dabei kann zudem im Betrieb durch mechanische Belastung der Streben durch einen Fluidgegendruck eine weitergehende Verformung über den Streckwinkel hinaus unter Ausnutzung der Elastizität der Strebenabschnitte und der Eigenelastizität des Gelenkmaterials möglich sein, wobei jedoch bei Erreichen des Streckwinkels die für eine weitergehende Verformung/Streckung notwendige Kraft steil ansteigt.

Das oder die Gelenke sind dabei vorteilhaft derart auf der Länge der einzelnen Streben angeordnet, dass sie verschiedene Abschnitte der Streben voneinander trennen und das Abwinkeln der einzelnen Abschnitte gegeneinander erlauben. Dadurch ist die Förderschaufel leicht faltbar oder einrollbar.

Vorteilhaft ist dabei wenigstens 1 Gelenk von einer Nabe des Rotors beabstandet, beispielsweise wenigstens um den Radius oder das Zweifache des Radius der Nabe.

Vorteilhaft sind alle oder wenigstens die Mehrzahl der Gelenke in derselben Bewegungsrichtung knickbar, so dass die gesamte Förderschaufel einrollbar ist.

Im eingerollten Zustand weist die Förderschaufel bzw. der Rotor, an dem sie befestigt ist, somit eine geringe radiale Ausdehnung auf und kann somit einfach an den Betriebsort gebracht werden.

Alternativ können benachbarte Gelenke auch zu entgegengesetzten Richungen abwinkelbar sein, um ein ziehharmonikaartiges Zusammenfalten zu ermöglichen. Wichtig dabei ist, dass durch den Fluidgegendruck im Betrieb alle Gelenke der Streben in eine definierte gestreckte Stellung gebracht und dort stabilisiert werden.

Nach dem Verbringen an den Betriebsort/Einsatzort kann der Rotor angetrieben werden, so dass die Förderschaufel oder mehrere Förderschaufeln durch Zentrifugalkräfte und/oder einen sich einstellenden Fluidgegendruck aufgerichtet werden.

In einer Abwandlung kann das Entfalten des Rotors auch durch elastische Rückstellkräfte in den Gelenken realisiert werden. Die elastischen Rückstellkräfte können auch in Kombination mit Zentrifugalkräften bzw. Fluidgegendruck wirksam werden.

Damit erhöht sich die aktive Fläche der Förderschaufel oder der Förderschaufeln, so dass im Betrieb die entsprechende Fluidpumpe einen guten Wirkungsgrad aufweist.

Um die jeweilige Förderschaufel im Einsatzzustand in expandierter Form zu stabilisieren, ist vorgesehen, dass die einzelnen Gelenke ein Überstrecken der Strebe bzw. der aneinander grenzenden Abschnitte über den Streckwinkel begrenzen. Dabei kann elastisch das jeweilige Gelenk auch einige Grad über 180°, jedoch vorteilhaft nicht mehr als 200° streckbar sein. Damit kann die Förderschaufel beispielsweise auf Stoßbelastungen im Betrieb mit elastischem Nachgeben reagieren.

Es sind vorteilhaft an jeder der Streben eine Mehrzahl von Gelenken, beispielsweise zwei, mehr als fünf, mehr als zehn oder mehr als zwanzig Gelenke vorgesehen, um ein geschmeidiges Aufrollen, Auffalten bzw. Komprimieren der Förderschaufeln zu erleichtern.

Die verschiedenen Streben können untereinander durch weitere Streben miteinander verbunden sein, die ein Aufspannen der Förderschaufel erleichtern und die Membran gespannt halten können. Auch diese Verbindungsstreben können ihrerseits mit Gelenken versehen sein.

Vorteilhaft können wenigstens zwei, insbesondere auch drei Streben von einer gemeinsamen Basis ausgehend parallel zueinander oder fächerförmig angeordnet sein. Damit lässt sich aus zwei oder mehr Streben leicht eine ergonomisch günstige Förderschaufel gestalten, die an einer Nabe eines Rotors befestigt sein kann. Die Förderschaufel kann insgesamt beispielsweise die Form eines Flugzeugpropellerblattes aufweisen.

Die einzelnen Streben können vorteilhaft im Bereich der Nabe von einem gemeinsamen Punkt ausgehen, die Förderschaufel radial nach außen fächerartig erweitern und gegebenenfalls zur Spitze der Förderschaufel radial nach außen hin wieder zusammenlaufen oder dort durch eine Querstrebe miteinander verbunden sein.

Es kann jedoch auch vorgesehen sein, dass die Streben im Bereich der Nabe an einem Bogensegment, beispielsweise einem Kreisbogensegment oder Ellipsenbogensegment oder einem Ring befestigt sind, das/der außen an der Nabe befestigt ist. Verschiedene Kreisbogensegmente von verschiedenen Förderschaufeln können dann zu einem Kreisring vereinigt sein, der auf eine Nabe eines Rotors leicht aufschiebbar ist.

Vorteilhaft kann zudem vorgesehen sein, dass die erste Bewegungsebene im maximal gestreckten Zustand der Gelenke, die einem expandierten Zustand des Rotors entspricht, parallel zu einer gemeinsamen Ebene der Streben oder im Bereich der Streben tangential zu der zwischen ihnen gespannten Membran verläuft. In diesem Fall kann die Förderschaufel leicht in der Ebene der Membran oder tangential zur Membranfläche zusammenklappbar oder -faltbar bzw. -rollbar sein und sich im komprimierten Zustand des Rotors beispielsweise in Umfangsrichtung um die Nabe aufwickeln lassen.

Alternativ dazu kann auch vorgesehen sein, dass die erste Bewegungsebene im maximal gestreckten Zustand der Gelenke, die einem expandierten Zustand des Rotors entspricht, im Wesentlichen senkrecht zu einer gemeinsamen Ebene der Streben oder im Bereich der Streben senkrecht zu der zwischen diesen gespannten Membran verläuft. In diesem Fall lässt sich jede der Förderschaufeln senkrecht zur Ebene der Schaufelfläche einrollen, abklappen oder falten, so dass die Förderschaufeln beispielsweise in Längsrichtung parallel zur Nabe an diese angelegt werden können, um die radiale Ausdehnung des Rotors zu minimieren.

Die einzelnen Gelenke verhindern jeweils in der dem Knicken entgegengesetzten Richtung eine entsprechende Kompressionsbewegung, so dass die Förderschaufeln im Betrieb stabilisiert sind.

Ein Zusammenfalten des jeweiligen Rotors kann beispielsweise dadurch geschehen oder unterstützt werden, dass der Rotor abgebremst oder entgegen der Betriebsrichtung beim Pumpen bewegt wird. Es kann vorgesehen sein, dass die Gelenke derart vorgespannt sind, dass sie nur durch den Fluidgegendruck bzw. die Zentrifugalkraft im Betrieb in gestrecktem Zustand gehalten werden, so dass der Rotor bzw. die Förderschaufeln sich im Stillstand des Rotors selbsttätig zusammenrollen.

Eine vorteilhafte Ausführungsform der Erfindung sieht vor, dass wenigstens ein Gelenk ein Stützelement aufweist, an dem wenigstens ein Abschnitt einer Strebe drehbar gelagert und zwei benachbarte Abschnitte im gestreckten Zustand abgestützt sind. Das jeweilige Stützelement kann beispielsweise als Platte mit einem Lagerbock ausgeführt sein, an dem jeweils ein Abschnitt einer Strebe derart gelagert ist, dass sie in einer ersten Richtung gegenüber dem Stützelement bzw. gegenüber einem zweiten Abschnitt der Strebe knickbar, in einer zweiten Bewegungsrichtung jedoch nur bis zu dem Streckwinkel, beispielsweise nicht wesentlich mehr als 180° in eine gestreckte Position der Strebe abwinkelbar ist. Dabei kann je nach Position des Lagerbocks bzw. der Lagerstelle der jeweilige Abschnitt entweder auf der dem zweiten Abschnitt zugewandten Seite des Lagers oder auf der dem zweiten Abschnitt abgewandten Seite des Lagers zur Begrenzung der Streckbewegung jeweils an dem Stützelement anschlagen.

Das Stützelement kann vorteilhaft symmetrisch bezüglich einer Mittelebene zwischen zwei an dem Gelenk aneinander angrenzenden Abschnitten aufgebaut sein. Das Stützelement kann zudem aus einem Werkstoff bestehen, der elastischer ist als der Werkstoff der Strebe.

Eine weitere vorteilhafte Ausgestaltung der Erfindung sieht vor, dass ein erster Abschnitt der Strebe an einem zweiten Abschnitt der Strebe in einem Lager eines Gelenks derart schwenkbar gelagert ist, dass die beiden Abschnitte in gestreckter Position teilweise in Längsrichtung überlappen und dass der erste Abschnitt einen Schwenkhebel auf einer ersten Seite des Lagers und einen Stützhebel auf der anderen Seite des Lagers aufweist, wobei der Stützhebel im gestreckten Zustand des Gelenkes an dem zweiten Abschnitt abgestützt ist.

Im gestreckten Zustand der Strebe liegen die beiden aneinander angrenzenden Abschnitte annähernd parallel zueinander und stützen sich aneinander ab, derart, dass der erste Abschnitt in einem an dem zweiten Abschnitt befestigten Lager gelagert ist und einen Schwenkhebel auf der einen Seite des Lagers und einen Stützhebel auf der gegenüberliegenden Seite des Lagers aufweist, wobei der Stützhebel sich im gestreckten Zustand an dem zweiten Abschnitt abstützt. In der entgegengesetzten Richtung ist der Schwenkhebel frei schwenkbar, und der Stützhebel entfernt sich von der Stelle, an der er in gestrecktem Zustand an dem zweiten Abschnitt abgestützt ist.

Alternativ dazu kann auch vorgesehen sein, dass ein erster Abschnitt der Strebe derart an einen zweiten Abschnitt der Strebe im Lager eines Gelenks gelagert ist, dass die beiden Abschnitte in gestreckter Position teilweise in Längsrichtung überlappen und dass der erste Abschnitt einen Schwenkhebel auf einer ersten Seite des Lagers und einen Stützhebel auf der zweiten Seite des Lagers aufweist, wobei der Schwenkhebel im gestreckten Zustand am Ende des zweiten Abschnittes abgestützt ist. In diesem Fall ist der Schwenkhebel seinerseits im gestreckten Zustand an dem zweiten Abschnitt abgestützt und in der entgegengesetzten Richtung frei bewegbar.

Eine weitere vorteilhafte Ausgestaltung der Erfindung sieht vor, dass ein erster Abschnitt der Strebe und ein zweiter Abschnitt der Strebe im gestreckten Zustand stirnseitig aneinander angeordnet und mittels eines asymmetrischen Filmgelenks miteinander verbunden sind.

Grundsätzlich sind durch das Vorsehen eines Filmgelenks die beiden Abschnitte gegeneinander bezüglich des zwischen ihnen eingeschlossenen Winkels abknickbar. Durch die asymmetrische Ausgestaltung des Filmgelenks wird sichergestellt, dass das Abknicken in einer ersten Richtung möglich ist, jedoch in einer zweiten Richtung im gestreckten Zustand der Strebe begrenzt wird.

Dazu kann konkret vorgesehen sein, dass das Filmgelenk auf der im komprimierten Zustand des Rotors und im geknickten Zustand der Strebe innen liegenden Seite der Strebe, dort, wo die gegeneinander geknickten Abschnitte der Strebe den kleineren Winkel miteinander einschließen, angeordnet ist und dass auf der außen liegenden Seite der Strebe jeder der Abschnitte einen Anschlag bildet, wobei die Anschläge im gestreckten Zustand der Strebe aneinander anliegen.

Mit der innen liegenden Seite der Strebe ist die Seite gemeint, die im komprimierten Zustand der Förderschaufel einen Winkel aufweist, der kleiner als 180° ist. In diesem Bereich können verschiedene aneinander angrenzende Abschnitte der Strebe aufeinander zugeklappt werden. Die entgegengesetzte Seite der Strebe in Bezug auf ihre Längsachse bzw. Längsebene wird äußere Seite genannt.

Ein entsprechendes Filmgelenk kann entweder aus einem anderen Werkstoff bestehen als die Abschnitte und mit beiden Abschnitten verbunden sein, beispielsweise durch Kleben, Schweißen oder andere Fügetechniken, oder das Filmgelenk kann aus demselben Werkstoff bestehen wie die Abschnitte und auch einstückig mit diesen zusammenhängen.

In diesem Fall ist es beispielsweise möglich, das Filmgelenk durch Einbringen einer Ausnehmung an der Außenseite der Strebe zu schaffen. Eine derartige Ausnehmung kann beispielsweise durch Laserschneiden, Ätzen oder andere Erosionstechniken eingebracht werden. Die entsprechende Ausnehmung kann als gerader Schnitt oder auch als keilförmige Ausnehmung oder in anderer Form eingebracht sein. Die Größe der Ausnehmung bestimmt darüber, wann bei einer Bewegung der Strebe in Streckrichtung die beiden Abschnitte an der dem Filmgelenk gegenüberliegenden äußeren Seite der Strebe aneinander anschlagen und damit eine Überstreckung begrenzen.

In einer anderen Alternative der Ausbildung eines Gelenks kann auch vorgesehen sein, dass der erste und der zweite Abschnitt der Strebe stirnseitig mittels eines Gelenkabschnittes miteinander verbunden sind, der auf der Innenseite der Strebe mindestens bis zur Mittelebene der Strebe aus einem Material besteht, das leichter komprimierbar ist als das Material auf der Außenseite der Strebe. Es ist dann ein gesonderter Gelenkabschnitt vorgesehen, der auf der Innen- und Außenseite der Strebe ungleich, beispielsweise aus verschiedenen Materialien bestehend, aufgebaut ist. Das Material auf der Innenseite der Strebe ist vorteilhaft leicht komprimierbar, jedenfalls leichter komprimierbar als auf der Außenseite der Strebe. Das auf der Außenseite der Strebe befindliche Material kann dehnbar, möglichst hart und nicht komprimierbar ausgestaltet sein. Das Material auf der Innenseite der Strebe kann bei guter Kompressibilität zugfest ausgebildet, beispielsweise durch zugfeste Fasern verstärkt sein.

Es kann auch vorgesehen sein, dass die Strebe im Gelenkbereich auf der Außenseite mit einem Material beschichtet ist, das härter ist als das Material des Gelenkabschnittes.

Die Erfindung bezieht sich außer auf eine Förderschaufel für einen komprimierbaren Rotor auch auf einen Rotor für eine Fluidpumpe, die mit entsprechenden Förderschaufeln versehen ist. Weiterhin bezieht sich die Erfindung auch auf eine entsprechende Fluidpumpe, insbesondere eine Blutpumpe für den medizinischen Bereich, die mit einem entsprechenden komprimierbaren Rotor bzw. komprimierbaren Förderschaufeln gemäß der Erfindung ausgestattet ist.

Im Folgenden wird die Erfindung anhand eines Ausführungsbeispiels in einer Zeichnung gezeigt und anschließend beschrieben. Dabei zeigt
- Fig. 1: grundsätzlich den Einsatz einer erfindungsgemäßen Blutpumpe mit einem komprimierbaren Rotor in einer Herzkammer,
- Fig. 2: schematisch den Aufbau einer Förderschaufel mit drei fächerförmig angeordneten Streben,
- Fig. 3: den Aufbau einer Förderschaufel mit einer kreissegmentförmigen Basis,
- Fig. 4: eine Ansicht eines Rotors in expandierter Form,
- Fig. 5: eine Ansicht des Rotors aus Fig. 4 in komprimierter Form,
- Fig. 6: eine Ansicht eines weiteren Rotors in expandierter Form,
- Fig. 7: eine Ansicht des Rotors aus Fig. 6 in komprimierter Form,
- Fig. 8: die schematische Ansicht eines Gelenks einer Strebe,

- Fig. 9: das Gelenk aus Fig. 8 in verschiedenen Zuständen schematisch,
- Fig. 10: ein weiteres Gelenk einer Strebe in einem ersten Zustand,
- Fig. 11: das Gelenk aus Fig. 10 in einem zweiten Zustand,
- Fig. 12: ein Gelenk einer Strebe, das in einem Gelenkabschnitt vorgesehen ist, an dem zwei Abschnitte stirnseitig angeordnet sind,
- Fig. 13: ein Filmgelenk einer Strebe in einem ersten Zustand,
- Fig. 14: das Filmgelenk aus Fig. 13 in einem anderen Zustand,
- Fig. 15: eine weitere Ausführungsform eines Filmgelenkes sowie
- Fig. 16: die Ausbildung eines Gelenkabschnittes an einer Strebe.

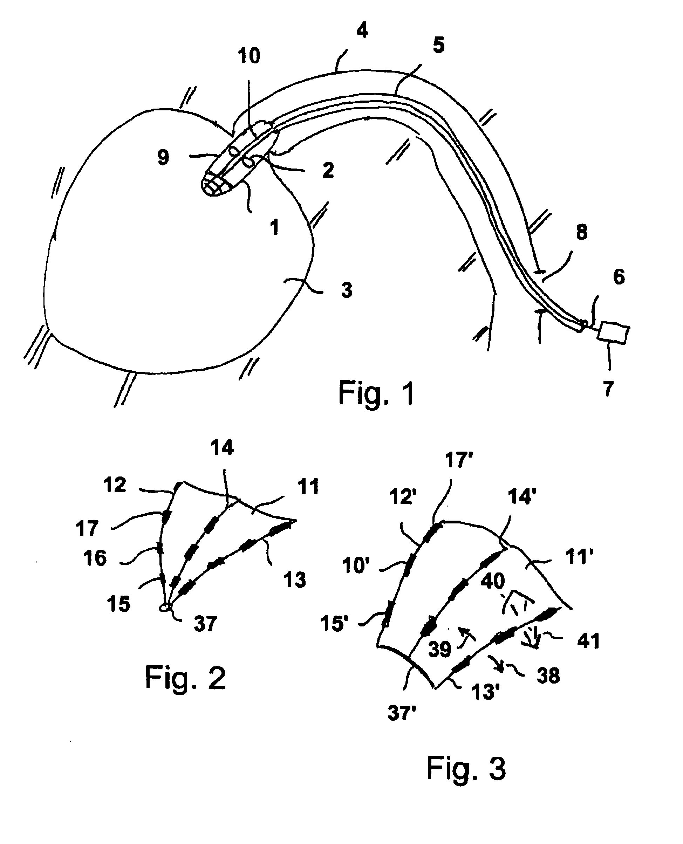

Fig. 1 zeigt eine an ihrem Einsatzort im Inneren einer Herzkammer 3 befindliche Herzpumpe 1 mit einem Rotor 2, der Förderschaufeln auf einer Nabe 10 aufweist und innerhalb eines Pumpengehäuses 9 angeordnet ist. Das Pumpengehäuse 9 befindet sich am Übergang von einem Blutgefäß 4 zur Herzkammer 3. Die Pumpe ist in der Lage, aus der Herzkammer 3 Blut anzusaugen und dies in das Blutgefäß 4 zu befördern.

Die Pumpe 1 ist am Ende eines Hohlkatheters 5 angeordnet, der durch eine Schleuse 8 in den Körper eines Patienten bzw. in das Blutgefäß 4 eingeführt ist und der in seinem Inneren eine mit hohen Drehzahlen antreibbare Welle 6 aufnimmt, die innerhalb der Pumpe mit der Nabe 10 verbunden ist. Die Welle 6 ist an ihrem antriebsseitigen, proximalen Ende mit einem Motor 7 verbunden.

Zum Transport der Pumpe 1 durch das Blutgefäß kann diese radial komprimiert werden, um dann nach dem Verbringen in die Herzkammer 3 radial expandiert zu werden und einen entsprechend verbesserten Wirkungsgrad bzw. die gewünschte Pumpleistung zu erzielen.

Fig. 2 zeigt beispielhaft eine erfindungsgemäße Förderschaufel 11 des Rotors 2, bei der zwischen drei Streben 12, 13, 14 eine Membran gespannt ist, die an den einzelnen Streben beispielsweise mittels Kleben, Schweißen oder auf ähnliche Weise befestigt ist.

Die Membran kann auch einfach durch Eintauchen der Streben in einen flüssigen Kunststoff, beispielsweise Polyurethan, aufgebracht werden. Die Streben 12, 13, 14 weisen jeweils eine Vielzahl von Gelenken 15, 16, 17 auf, von denen an den einzelnen Streben drei bzw. vier dargestellt sind.

Die Streben 12, 13, 14 laufen an ihrer Basis in einem Punkt 37 zusammen, in dem sie an einer Nabe 10 befestigt sind.

Auf die Natur der Gelenke 15, 16, 17 wird weiter unten noch genauer eingegangen.

Die Fig. 3 zeigt eine abgewandelte Bauform einer Förderschaufel 11' mit Streben 12', 13', 14', die jeweils Gelenke 15', 16', 17' aufweisen und die an einer Basis 37' zusammenlaufen, die kreisbogensegmentförmig ausgestaltet ist und an einer entsprechend geformten Nabe 10 eines Rotors befestigt werden kann.

Mit den Pfeilen 38, 39 ist in der Fig. 3 die Bewegungsebene dargestellt, die innerhalb der Ebene der Streben 12', 13', 14' bzw. tangential zu der Membran im jeweiligen Punkt ausgerichtet ist.

Mit den Pfeilen 40, 41 sind die Richtungen senkrecht zu der entsprechenden Ebene der Membran bzw. der Tangentialfläche der Membran im jeweiligen Punkt angedeutet.

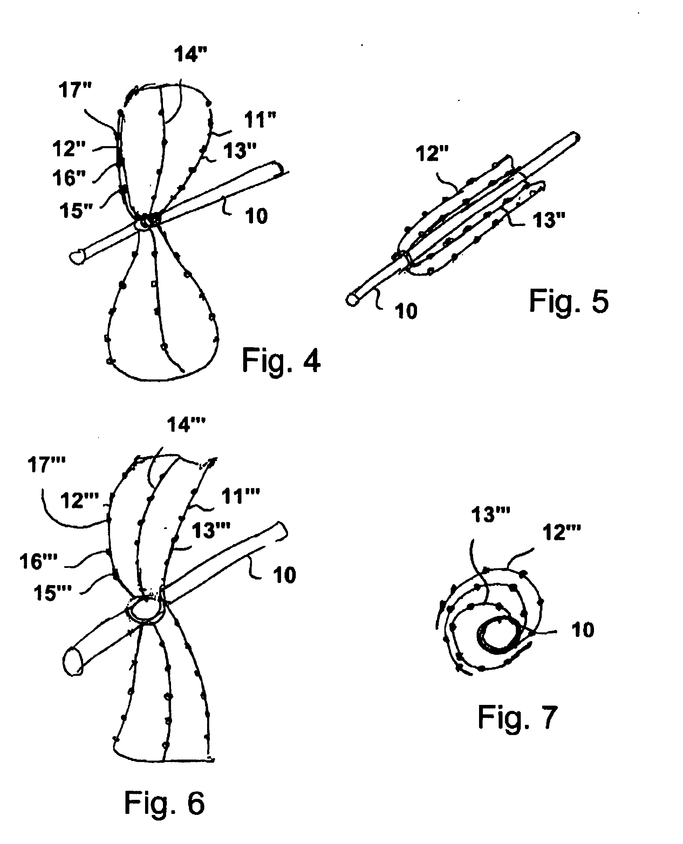

Die Fig. 4 zeigt zwei Förderschaufeln, von denen die obere mit 11" bezeichnet und näher dargestellt ist. Die beiden Förderschaufeln sind an einer Nabe 10 einander radial gegenüberliegend symmetrisch befestigt. Die Förderschaufel 11" weist Streben 12 " , 13'' auf, zwischen denen eine Membran gespannt ist, wobei jede der Streben sowie eine zwischen ihnen liegende dritte Strebe 14'' Gelenke 15", 16 " , 17 " aufweist, die in der Ebene senkrecht zur Fläche der Förderschaufel 11 " bzw. an den jeweiligen Streben senkrecht zur Membranfläche bzw. der Tangente der Membranfläche knickbar sind.

Auf diese Weise können die einzelnen Förderschaufeln 11 " in axialer Richtung der Nabe 10 an diese angeklappt werden, wie dies in der Fig. 5 im komprimierten Zustand des Rotors anhand der Streben 12 " und 13 " dargestellt ist.

Durch Rotation der Nabe, angetrieben durch die in der Fig. 1 dargestellte Welle 6, richten sich die Streben 12", 13 " durch Zentrifugalkräfte im Betrieb radial auf.

Die Fig. 6 zeigt einen weiteren Rotor mit zwei Förderschaufeln, von denen die obere mit 11''' bezeichnet ist. Diese weist Streben 12''', 13''', 14''' auf, die jeweils mit Gelenken 15''', 16''', 17''' versehen sind. Die Gelenke sind jeweils in einer ersten Bewegungsrichtung innerhalb der Ebene der Förderschaufel, d. h. parallel zur Membran im Bereich der jeweiligen Strebe bzw. einer Tangentialfläche der Membran, knickbar, so dass die Förderschaufeln 11''' in Umfangsrichtung an die Nabe 10 anklappbar bzw. - faltbar sind.

Im komprimierten Zustand legen sich die Streben 12''', 13''' um die Nabe 10 herum, wie in der Fig. 7 näher dargestellt ist.

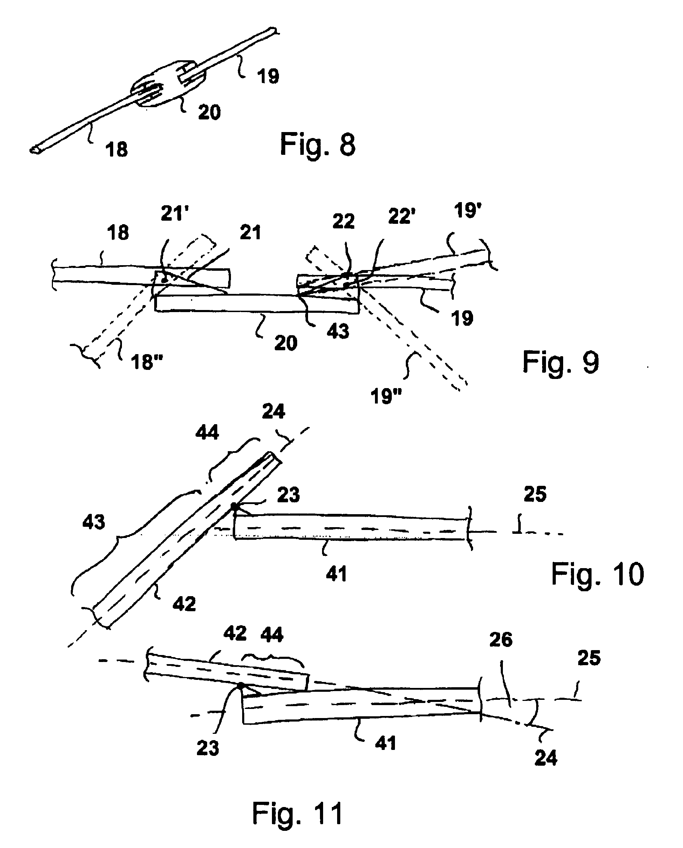

Die Fig. 8 zeigt genauer die Konstruktion der Gelenke in einer ersten Ausführungsform, wobei ein Stützelement 20 in Form einer Platte zwischen zwei Abschnitten 18, 19 einer Strebe dargestellt ist. Das Stützelement 20 weist zwei Lagerböcke 21, 22 auf, die in der Fig. 9 näher in einer Seitenansicht dargestellt sind und in denen jeweils eine Lagerwelle 21', 22' gelagert ist. Auf den entsprechenden Wellen sind die Abschnitte 18, 19 drehbar gelagert.

Auf der rechten Seite des Stützelementes 20 ist der Abschnitt 19 zusätzlich gestrichelt in der überstreckten Position 19' dargestellt, in der der Abschnitt sich an der mit 43 bezeichneten Stelle an dem Stützelement 20 abstützt, wodurch eine weitere Abwinklung des Abschnittes 19 gegenüber dem Abschnitt 18 verhindert wird.

Auf der rechten Seite ist mit 19'' der Abschnitt 19 in der abgeknickten Position bezeichnet, die ebenfalls gestrichelt dargestellt ist. In dieser abgeknickten Lage ist die entsprechende Strebe mit den Abschnitten 18, 19 abgewinkelt bzw. gefaltet, so dass der Rotor eine komprimierte Position einnimmt.

Auf der linken Seite ist mit 18'' nur die abgewinkelte Position des Abschnitts 18 dargestellt.

Die Fig. 10 stellt eine weitere Ausführungsform der Erfindung dar, bei der auf ein Stützelement verzichtet wird, die beiden Abschnitte 41, 42 einander in gestreckter Position teilweise in Längsrichtung überdecken, wobei der Abschnitt 42 einen Schwenkhebel 43 und einen Stützhebel 44 zu den beiden Seiten des Lagerpunktes 23 aufweist und wobei der Schwenkhebel 43 über den anderen Abschnitt 41 hinausragt und abwinkelbar ist.

In der Fig. 11 ist die Anordnung mit den beiden Abschnitten 41, 42 in einer maximal gestreckten Position dargestellt, wobei der gegenüber der Fig. 10 geschwenkte Abschnitt 42' in der maximal überstreckten Position dargestellt ist. Der Stützhebel 44 stützt sich in dieser Position gegen den Abschnitt 41 ab. Damit ist ein weiteres Überstrecken der Strebe, die die beiden Abschnitte 41, 42 aufweist, verhindert.

Wichtig für eine solche Ausgestaltung eines Gelenks ist, dass die Längsachsen 24, 25 bzw. die Schwenkebenen der beiden Abschnitte 42, 41 sich in derselben Ebene bzw. in parallelen Ebenen, die nur minimal gegeneinander versetzt sind, befinden.

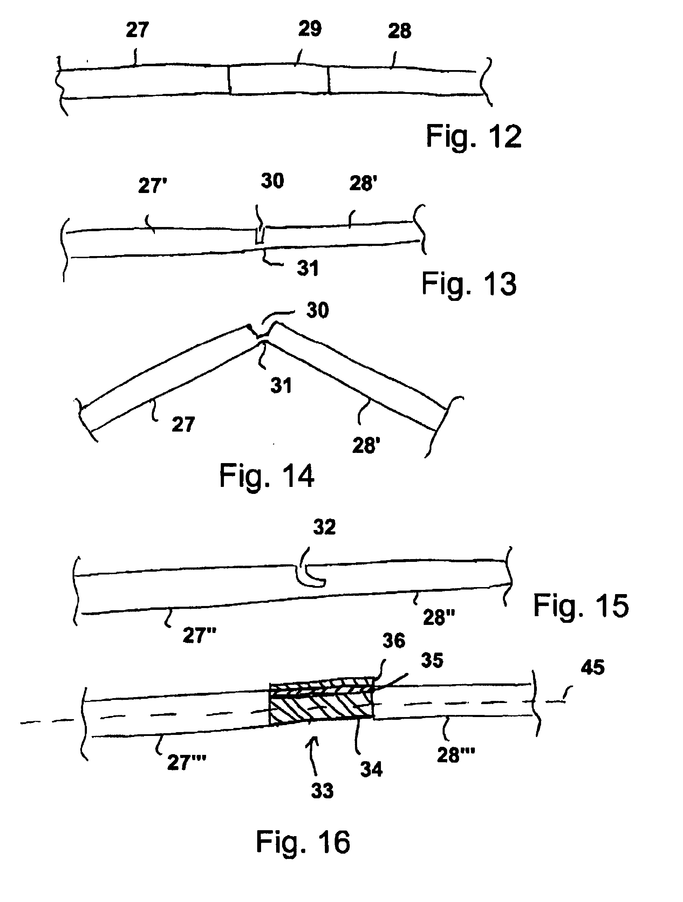

In der Fig. 12 ist schematisch eine Strebe mit zwei Abschnitten 27, 28 dargestellt, die durch einen Gelenkabschnitt 29 miteinander verbunden sind.

Die Fig. 13 zeigt konkreter eine Ausnehmung 30 in Form eines Schlitzes bzw. Schnittes, der zwischen den Abschnitten 27', 28' in die Strebe eingebracht ist.

Die Fig. 14 zeigt die Strebe aus Fig. 13 in einer geknickten Anordnung der Abschnitte 27', 28', wobei das Filmgelenk 31 auf der Innenseite der Strebe und die Ausnehmung 30 auf deren Außenseite liegt. Die Abschnitte 27', 28' schließen somit auf der Innenseite einen kleineren Winkel ein als auf der Außenseite, wenn die Strebe abgewinkelt ist.

Im gestreckten Zustand der Strebe weisen die Abschnitte auf der Innenseite einen Winkel auf, der maximal 180° oder nur wenig darüber, beispielsweise maximal 190°, beträgt.

In der Fig. 15 ist eine andere Form der Ausnehmung 32 dargestellt, die keinem geraden Schnitt, sondern einer komplizierteren Form folgt und die damit ein längeres und flexibleres Filmgelenk schafft. Eine derartige, komplexe Ausnehmung 32 kann beispielsweise mittels Laserschneiden oder Ätztechniken oder anderer erosiver Verarbeitungstechniken eingebracht werden.

Fig. 16 stellt eine andere Alternative bei der Bildung eines Gelenks an einer Strebe dar. In der Fig. 16 ist der Gelenkabschnitt zwischen zwei Abschnitten 27''' und 28''' mit 33 bezeichnet. Dieser Gelenkabschnitt 33 weist auf der Innenseite ein Material 34 auf, das sich auch über die Mittelebene 45 der entsprechenden Strebe, die senkrecht zur Zeichenebene verläuft, hinaus bis zur Außenseite der Strebe erstrecken kann.

Vorteilhaft ist auf der Außenseite der Strebe eine Schicht 35 vorgesehen, die härter als das Material 34 und vor allem inkompressibel ist, so dass die Strebe nicht zur Außenseite abgewinkelt werden kann und durch die Beschaffenheit des Materials des Teils 35 bereits das Überstrecken der Strebe verhindert wird. Das Material 34 ist vorteilhaft leicht kompressibel, jedoch fest.

Zudem ist in der Fig. 16 eine Schicht 36 dargestellt, die ebenfalls anstelle der Schicht 35 oder zusätzlich zu dieser an der Außenseite der Strebe aufgebracht sein kann, jedoch ebenso wie das Material der Schicht 35 dehnbar ist, damit einerseits ein Knicken der Abschnitte 27''', 28''' zur Innenseite der Strebe ermöglicht wird, ein Abknicken der Abschnitte zur Außenseite jedoch begrenzt wird.

Durch die erfindungsgemäße Gestaltung von Förderschaufeln bzw. einem Rotor für eine Fluidpumpe mit den entsprechenden Gelenken wird eine einfache Komprimierbarkeit eines Rotors für eine Fluidpumpe erreicht, so dass die Förderschaufeln entweder ganz ohne Gegenkräfte oder mit geringen elastischen Gegenkräften in den komprimierten Zustand bringbar und nach dem Verbringen an den Betriebsort auch wieder aufstellbar sind. Die beschriebenen Gelenke sind einfach ausgeführt, einfach herstellbar und zuverlässig und verleihen den entsprechenden Förderschaufeln eine hohe Flexibilität.

## Patentansprüche

1. Förderschaufel für einen radial komprimierbaren und expandierbaren Rotor (2) einer Fluidpumpe mit wenigstens zwei Streben (12, 12', 12'', 12''', 13, 13', 13'', 13''', 14, 14', 14'', 14''', 18, 19, 41, 42, 27, 27', 27'', 28, 28', 28'') und einer im expandierten Zustand von diesen gehaltenen Membran, **dadurch gekennzeichnet, dass** die Streben jeweils wenigstens ein, insbesondere mehr als ein Gelenk (15, 15', 16, 16', 17, 17', 15''', 16''', 17''', 21, 21', 22, 22', 23) aufweisen, das in einer ersten Bewegungsebene in einer ersten Richtung ein Abwinkeln ermöglicht und in der entgegengesetzten zweiten Richtung innerhalb der ersten Bewegungsebene ein Überstrecken der Strebe über einen Streckwinkel begrenzt, der insbesondere 180° beträgt.

2. Förderschaufel nach Anspruch 1, **dadurch gekennzeichnet, dass** wenigstens 1 Gelenk von einer Nabe des Rotors beabstandet ist.

3. Förderschaufel nach Anspruch 1 oder 2, **dadurch gekennzeichnet, dass** wenigstens zwei der Streben (12, 12', 12'', 12''', 13, 13', 13'', 13''', 14, 14', 14'', 14''', 18, 19, 41, 42, 27, 27', 27'', 28, 28', 28''), insbesondere drei Streben, von einer gemeinsamen Basis ausgehend parallel zueinander oder fächerförmig angeordnet sind.

4. Förderschaufel nach Anspruch 2 oder 3, **dadurch gekennzeichnet, dass** die Basis (37) ein Punkt ist.

5. Förderschaufel nach Anspruch 2 oder 3, **dadurch gekennzeichnet, dass** die Basis (37') ein Bogensegment ist.

6. Förderschaufel nach Anspruch 1 oder einem der folgenden, **dadurch gekennzeichnet, dass** einander benachbarte Gelenke einer Strebe in entgegengesetzte Richtungen abwinkelbar sind.

7. Förderschaufel nach Anspruch 1 oder einem der folgenden, **dadurch gekennzeichnet, dass** die erste Bewegungsebene im maximal gestreckten Zustand der Gelenke, die einem expandierten Zustand des Rotors entspricht, parallel zu einer gemeinsamen Ebene der Streben oder im Bereich der Streben tangential zu der zwischen ihnen gespannten Membran verläuft.

8. Förderschaufel nach einem der Ansprüche 1 bis 6, **dadurch gekennzeichnet, dass** die erste Bewegungsebene im maximal gestreckten Zustand der Gelenke, die einem expandierten Zustand des Rotors entspricht, im Wesentlichen senkrecht zu einer gemeinsamen Ebene der Streben oder im Bereich der Streben senkrecht zu der zwischen diesen gespannten Membran verläuft.

9. Förderschaufel nach einem der Ansprüche 1 bis 8, **dadurch gekennzeichnet, dass** wenigstens ein Gelenk ein Stützelement (20) aufweist, an dem wenigstens ein Abschnitt (19, 20) einer Strebe drehbar gelagert und zwei benachbarte Abschnitte im gestreckten Zustand abgestützt sind.

10. Förderschaufel nach einem der Ansprüche 1 bis 9, **dadurch gekennzeichnet, dass** ein erster Abschnitt (42) der Strebe an einem zweiten Abschnitt (41) der Strebe in einem Lager eines Gelenks (23) derart schwenkbar gelagert ist, dass die beiden Abschnitte in gestreckter Position teilweise in Längsrichtung überlappen und dass der erste Abschnitt einen Schwenkhebel (43) auf einer ersten Seite des Lagers und einen Stützhebel (44) auf der zweiten Seite des Lagers aufweist, wobei der Stützhebel im gestreckten Zustand des Gelenkes an dem zweiten Abschnitt (41) abgestützt ist.

11. Förderschaufel nach Anspruch 1 oder einem der folgenden, **dadurch gekennzeichnet, dass** ein erster Abschnitt (42) der Strebe derart an einen zweiten Abschnitt (41) der Strebe im Lager eines Gelenks gelagert ist, dass die beiden Abschnitte in gestreckter Position teilweise in Längsrichtung überlappen und dass der erste Abschnitt (42) einen Schwenkhebel (43) auf einer ersten Seite des Lagers und einen Stützhebel (44) auf der zweiten Seite des Lagers aufweist, wobei der Schwenkhebel im gestreckten Zustand am Ende des zweiten Abschnittes abgestützt ist.

12. Förderschaufel nach Anspruch 1 oder einem der folgenden, **dadurch gekennzeichnet, dass** ein erster Abschnitt (27, 27', 27'', 27''') der Strebe und ein zweiter Abschnitt (28, 28', 28'', 28''') der Strebe im gestreckten Zustand stirnseitig aneinander angeordnet und mittels eines asymmetrischen Filmgelenks (31) miteinander verbunden sind.

13. Förderschaufel nach Anspruch 12, **dadurch gekennzeichnet, dass** das Filmgelenk (31) auf der im komprimierten Zustand des Rotors und im geknickten Zustand der Strebe auf der innen liegenden Seite der Strebe, dort, wo die gegeneinander geknickten Abschnitte (27, 27', 27'', 27''', 28, 28', 28'', 28''') der Strebe den kleineren Winkel miteinander einschließen, angeordnet ist und dass auf der außen liegenden Seite der Strebe jeder der Abschnitte einen Anschlag bildet, wobei die Anschläge im gestreckten Zustand der Strebe aneinander anliegen.

14. Förderschaufel nach Anspruch 13, **dadurch gekennzeichnet, dass** das Filmgelenk (31) durch eine Ausnehmung (30, 32) an der Außenseite der Strebe gebildet ist.

15. Förderschaufel nach Anspruch 13, **dadurch gekennzeichnet, dass** der erste und der zweite Abschnitt (27, 27', 27'', 27''', 28, 28', 28'', 28''') der Strebe stirnseitig mittels eines Gelenkabschnittes (29) miteinander verbunden sind, der auf der Innenseite der Strebe mindestens bis zur Mittelebene (45) der Strebe aus einem Material besteht, das leichter komprimierbar ist als das Material auf der Außenseite der Strebe.

16. Förderschaufel nach Anspruch 15, **dadurch gekennzeichnet, dass** das Material auf der Innenseite kompressibler als auf der Außenseite, aber zugfest ist.

17. Förderschaufel nach Anspruch 15 oder 16, **dadurch gekennzeichnet, dass** die Strebe im Gelenkbereich auf der Außenseite mit einem Material (36) beschichtet ist, das härter ist als das Material des Gelenkabschnittes.

18. Rotor für eine Fluidpumpe, insbesondere eine Herzpumpe, mit wenigstens einer, insbesondere mehreren Förderschaufeln gemäß einem der Ansprüche 1 bis 17.
